# EUROPÄISCHE PATENTANMELDUNG

(11) **EP 4 356 755 A1**
(43) Veröffentlichungstag der Anmeldung: **24.04.2024**
(21) Anmeldenummer: 22202893.8
(22) Anmeldetag: 21.10.2022
(51) Int. Cl.: A23L 11/50, A23C 11/10, A23J 1/14, A23J 3/14, A23J 3/34, A23L 33/18, A23L 33/185

(54) **FERMENTIERTE PFLANZENPROTEINE**

(71) Anmelder: DMK Deutsches Milchkontor GmbH, 27404 Zeven (DE)
(72) Erfinder: STADLER, Christian, 27404 Zeven (DE); SIEDENTOPP, Chris, 27404 Zeven (DE); SAGURSKI, Arthur, 26188 Edewecht (DE); POMMER, Jan, 26188 Edewecht (DE)
(74) Vertreter: Fabry, Bernd

(57) **Zusammenfassung**

Vorgeschlagen werden fermentierte Pflanzenproteine, erhältlich oder erhalten durch die folgenden Schritte
(a) Bereitstellen einer pflanzlichen Proteinquelle;
(b) Versetzen der Proteine aus Schritt (a) mit Wasser unter Erhalt einer wässrigen Proteinphase;
(c) Versetzen der wässrigen Proteinphase aus Schritt (b) mit Mikroorganismen, die zur Fermentation von Proteinen geeignet sind, und/oder Proteasen; und
(d) Fermentierung der wässrigen Proteinphase aus Schritt (c) sowie gegebenenfalls
(e) Inaktivierung und Abtrennung der Mikroorganismen und/oder Enzyme.
und ihre Verwendung in veganen Milchersatzprodukten.

## Beschreibung

### GEBIET DER ERFINDUNG

Die vorliegende Erfindung befindet sich auf dem Gebiet der veganen Lebensmittel und betrifft fermentierte Pflanzenproteine, ein Verfahren zu deren Herstellung sowie deren Verwendung zur Herstellung von veganen Milchersatzprodukten.

### TECHNOLOGISCHER HINTERGRUND

Unter veganen Lebensmitteln versteht man für die menschliche Ernährung vorgesehene Produkte, die ausschließlich pflanzenbasiert sind und beispielsweise Gemüse, Obst, Früchte und Nüsse sowie daraus hergestellte Zubereitungen umfassen. Der Trend zu veganer Ernährung hat in den letzten 10 Jahren stark zugenommen. 2021 ernährten sich laut Bundesministerium für Ernährung und Landwirtschaft (BMEL) 10 % der deutschen Bevölkerung vegetarisch und 2 % vegan - jeweils doppelt so viele wie noch im Jahr zuvor! Seit 2017 liegt der Anteil der Neueinführung von veganen Lebensmitteln und Getränke in Deutschland bei 15 %.

Der Trend zu veganen Alternativen geht daher auch nicht an der milchverarbeitenden Industrie vorbei. Laut einer Publikation von Future Grocery Shopping kaufen in Deutschland mittlerweile sogar 93 % der Verbraucher regelmäßig pflanzliche Milchalternativen. Der Umsatz stieg zwischen 2018 und 2020 um 42 %. Dieser ist vor allem auf die wachsende Beliebtheit von Hafermilch zurückzuführen. Ungeachtet des sich stark entwickelnden Marktes sind Milchersatzprodukte, wie vegane Milch oder veganer Käse in ihrer Sensorik - Geschmack, Textur und Farbe - oftmals noch weit vom Original entfernt. Erwähnt seien beispielsweise der typische Bittergeschmack vieler pflanzlicher Proteine, die zur Herstellung der Ersatzprodukte erforderlich sind sowie die Vielzahl von Hilfs- und Zusatzstoffen, die für die Nachahmung einer entsprechenden Textur erforderlich sind und deren Menge vermindert werden sollte.

### STAND DER TECHNIK

Gegenstand der WO 2020 089383 A1 ist ein veganer Käseersatz, welcher auf Basis von Ballaststoffen, Calcium, Lipiden und Pflanzenprotein hergestellt wird.

Das Dokument EP 3213638 A1 offenbar ein Käseanalogon, welches Wasser, eine Wurzel- oder Knollenstärke, natürliches Kartoffelprotein und eine Fettkomponente umfasst.

### AUFGABE DER ERFINDUNG

Daher hat die Aufgabe der vorliegenden Erfindung darin bestanden, die Defizite, die pflanzliche Proteine hinsichtlich Geschmackes, Textur und Farbe aufweisen, zu beheben und vegane proteinhaltige Milchersatzprodukte mit entsprechend verbesserten sensorischen Eigenschaften zur Verfügung zu stellen.

### BESCHREIBUNG DER ERFINDUNG

Ein erster Gegenstand der Erfindung betrifft fermentierte Pflanzenproteine, erhältlich oder erhalten durch die folgenden Schritte
(a) Bereitstellen einer pflanzlichen Proteinquelle;
(b) Versetzen der Proteine aus Schritt (a) mit Wasser unter Erhalt einer wässrigen Proteinphase;
(c) Versetzen der wässrigen Proteinphase aus Schritt (b) mit Mikroorganismen und/oder Enzymen, die zur Fermentation von Proteinen geeignet sind; und
(d) Fermentierung der wässrigen Proteinphase aus Schritt (c) sowie gegebenenfalls
(e) Inaktivierung und Abtrennung der Mikroorganismen und/oder Enzyme.

Ein weiterer Gegenstand der Erfindung betrifft ein entsprechendes Verfahren zur Herstellung fermentierter Pflanzenproteine, umfassend oder bestehend aus den folgenden Schritten:
(a) Bereitstellen einer pflanzlichen Proteinquelle;
(b) Versetzen der Proteine aus Schritt (a) mit Wasser unter Erhalt einer wässrigen Proteinphase;
(c) Versetzen der wässrigen Proteinphase aus Schritt (b) mit Mikroorganismen und/oder Enzymen, die zur Fermentation von Proteinen geeignet sind; und
(d) Fermentierung der wässrigen Proteinphase aus Schritt (c) sowie gegebenenfalls
(e) Inaktivierung und Abtrennung der Mikroorganismen und/oder Enzyme.

Überraschenderweise wurde gefunden, dass fermentierte Pflanzenproteine über deutlich verbesserte Eigenschaften verfügen und sich mit ihnen vegane Milchersatzprodukte konfektionieren lassen, die in Geschmack, Textur und Farbe den milchbasierten Produkten kaum noch nachstehen.

Bei der Fermentation entsprechend der vorliegenden Erfindung werden folgende Veränderungen in den Eigenschaften der Pflanzenproteine beobachtet:
- **Farbe:** Insbesondere nach einer Vorbehandlung der Proteine (z.B. Erhitzung, Heißhaltung oder Hochdruckbehandlung) wird eine Aufhellung der Farbe beobachtet;
- **Geschmack:** Es werden bestimmte Aromen gebildet, unerwünschte sensorischen Eigenschaften wie beispielsweise der typische Bittergeschmack der Pflanzenproteine werden neutralisiert, arttypischer Geschmack wie bspw. "bohnig, erbsig, erdig, pilzig" wird reduziert. Hierdurch kann der Einsatz von Geschmacksverstärkern beispielsweise in Form von Hefeextrakten vermieden oder reduziert werden, Es wird ferner eine geschmackliche Säurenote gebildet die dem sensorischen Profil von konventionellen Milchprodukten ähnelt, und im Vergleich mit einem Säuerungsmittel wie z.B. Zitronensäure nicht hervorgehoben werden kann;
- **Geruch:** Im Geruch des Endproduktes wird durch die Fermentation eine natürliche Säurenote hervorgehoben, die den Bedarf von Aromen und Säuerungsmitteln reduziert und somit dem konventionellen Milchprodukt näherkommt;
- **Konsistenz:** Mit der Fermentation resultiert in den veganen Milchersatzprodukten eine cremige Konsistenz, die über den Einsatz zusätzlicher Zusatzstoffe oder herkömmlicher Prozesstechnologie schwer erreicht werden kann. Auf diese Weise wird auch ein längeres Mundgefühl erzeugt. Unerwünschte sensorische Eigenschaften wie: "klebrig, mehlig, talgig, grießig, schmierig, kurz, brüchig und fest" werden vermindert oder ganz vermieden;
- **Beschaffenheit:** Beim Herstellungsprozess wird die Viskosität beeinflusst was sich bei der Verarbeitung vorteilhaft erweist und auch letztlich im Endprodukt zu einer verbesserten Funktion führt. Die Wasser- und Ölbindung wird verbessert, wodurch die erforderliche Menge an Emulgatoren im Endprodukt reduziert werden kann.
- **Eigenschaften im Endprodukt:** Strukturbildung bei der Anwendung in Form von Elastizität, Ziehelastizität, Wasserbindung, Ölbindung, Gas-/Lochbildung, Aufschäumbarkeit (Stabilisierung Luftblasen). Geschmacksbildung durch Stoffwechsel entstehen Aromen, wodurch Geschmacksverstärker und/oder weiterer Einsatz von Aromen reduziert werden kann.

### Pflanzenproteine

Pflanzliche Proteine im Sinne der Erfindung können aus Ackerbohnen, Hafer, Reis, Mandeln, Eicheln, Kartoffeln, Soja, Erbsen, Lupinen, Raps oder anderen proteinreichen Früchten wie z. B. Sonnenblumen- oder Kürbiskernen gewonnen werden.

Die geernteten proteinhaltigen Früchte werden mechanisch zerkleinert und entfettet. Es entstehen Flocken oder ein proteinreiches Pulver. Anschließend wird unter Verwendung von Lösungsmitteln ein Proteinkonzentrat gewonnen, das ggf. weiter zu Proteinisolat gereinigt und aufkonzentriert wird: Die Flocken oder Mehl werden mit Wasser versetzt und angemaischt. Die proteinarmen Fasern und Feststoffe werden im nächsten Schritt mit Hilfe von Industriezentrifugen von der proteinreichen Lösung abgetrennt. Dann folgt die sogenannte Ausfällung. Hier wird der pH-Wert der proteinreichen Lösung auf den isoelektrischen Punkt eingestellt. Dadurch setzen sich die Proteinpartikel ab. Diese werden dann wiederum mittels Zentrifugen von der Lauge abgetrennt. Um alle Bestandteile der Mutterlauge aus dem ausgefällten und abgetrennten Protein zu entfernen, wird das Protein erneut mit Wasser versetzt und wieder mit Hilfe der Zentrifugalkraft abgetrennt. Bei einer Trockenextrusion wird unter Zuführung von Wärme, Druck und Hilfsstoffen ein Zwischenprodukt mit niedrigem Wassergehalt erzeugt. Diese ebenfalls geeigneten Proteine werden als TVP (Texturized Vegetable Protein) bezeichnet und haben eine trockene Konsistenz in Form von Körnern, Streifen oder Flocken. Bei einer Nassextrusion wird alternativ mit einem höheren Wassergehalt operiert. Die Feuchtigkeit des Zwischenproduktes liegt daher näher am Wassergehalt des Endproduktes. Das Zwischenprodukt wird als HMMA (High Moistured Meat Analogues) bezeichnet.

Die Proteine können in allen Ausgangsvariationen, nämlich Rohform geschält-gemahlen, Rohform geschält-gemahlen-luftsepariert, Rohform geschält-gemahlen-konzentriert oder als Proteinisolat eingesetzt werden.

Als besonders vorteilhaft hat es sich erwiesen, die Proteine erhöhtem Druck im Bereich von etwa 20 bis etwa 250 bar und/oder erhöhter Temperatur von etwa 75 bis etwa 95 °C mit einer Heißhaltezeit von 5 - 10min. auszusetzen und so vorzubehandeln. Da hierdurch Proteine optimal dispergiert, eine Sedimentation während der Fermentation somit reduziert und die bakteriologischen Risiken weitestgehend minimiert werden.

Im weiteren Verlauf des Verfahrens werden die gegebenenfalls vorbehandelten Proteine in Wasser welches wahlweise auch mit Salzen wie bspw. Natriumchlorid, Natriumcitrat, Calciumcitrat, Natriumphosphat, Calciumphosphat gelöst bzw. dispergiert, wobei es bevorzugt ist eine Trockenmasse von etwa 3 bis etwa 25 Gew.-% und insbesondere von etwa 3 bis etwa 20 Gew.-% einzustellen. Hierdurch ist eine Stabilisierung der Proteine während der Erhitzung und ein Teil der Salzkonzentration im weiterverarbeiteten Prozess sichergestellt.

### Pflanzenfette und Ballaststoffe

In einer bevorzugten Ausführungsform können der wässrigen Proteinphase aus Schritt (b) mindestens ein Pflanzenfett und/oder mindestens einen Ballaststoff zugesetzt werden, wobei die bevorzugten Mengen jeweils etwa 10 bis etwa 25 Gew.-% und insbesondere etwa 2 bis etwa 10 Gew.-% betragen. Wahlweise könnte jedoch die Fermentation auch ohne Einsatz von Pflanzenfetten oder Ballaststoffen erfolgen. Neben den geschmacklichen, farblichen, anwendungstechnischen und konsistenzbeeinflussenden Veränderungen ist der Einsatz von pflanzlichen Fetten von Vorteil, da es hierbei zu weniger starken Anbrennungen bzw. Biofouling in den Erhitzeranlagen kommt.

Geeignete Pflanzenfette sind ausgewählt aus der Gruppe, die gebildet wird von Kakaobutter, Kokosöl, Palmöl, Palmkernöl, Kokosfett, Nussöl, Sheabutter, Aprikosenöl, Sojalecithin, Sonnenblumenöl, Sojaöl, Rapsöl, Nussöl oder Mischungen davon. Durch die Zugabe der Fette werden die Proteine im gesamten Prozess gegen thermische Belastung und Zersetzung stabilisiert. Die Fette können daher auch schon auf der Stufe der Druck/Temperaturvorbehandlung zugegeben werden. Sie haben weiterhin die Funktion, die Aromen zu binden, die während der Fermentation entstehen.

Geeignete Ballaststoffe sind Pflanzenfasern beispielsweise ausgewählt aus der Gruppe, die gebildet wird von Citrus-, Kokosnuss-, Dinkel-, Hafer- und Apfelfasern. Diese haben bei der Fermentation strukturgebende, sensorische und anwendungstechnische Vorteile ergeben, da diese Ausgangsstoffe einen Eintrag von Ballaststoffen und Kohlenhydrate mit sich bringen. Die veganen Milchersatzprodukte weisen anschließend einen vollmundigeren Geschmack auf und benötigen geringere Mengen an Emulgatoren, Stabilisatoren, sowie Substrate, die für die Stoffwechselaktivität benötigt werden.

### Mikroorganismen

Mikroorganismen, die geeignet sind, Proteine zu fermentieren, sind beispielsweise ausgewählt aus der folgenden Gruppe: mesophile als auch thermophile Mikroorganismen wie bspw. stammend aus den Gattungen Streptokokken (z.B. Sc. Thermophilus, etc.), Lactokokken (z.B. Lc. Lactis, Lc. Cremoris, Lc. Diacetylactis, etc.), Lactobacillen (z.B. Lb. Acidophilus, Lb. Bulgaricus, etc.), Leuconostoc (z.B. Leuc. Cremoris, etc.), Bifidobacterien (z.B. Bifidum) und deren Kombinationen daraus.

### Enzyme

Geeignete proteolytische Enzyme, die auch Proteasen oder Peptidasen bezeichnet werden umfassen Aspartylproteasen (z.B. Pepsin, Chymosin, Cathepsin E), Cysteinprotease (z.B. Papain, Cathepsin K, Caspase, Calpain), Serinproteasen (z.B. Chymotropsin, Plasmin, Thrombin, Trypsin, Granzyme, Kallikrein) sowie Threonylproteasen (z.B. Proteasom) und deren Mischungen. Die einzelnen Proteasen weisen bestimmte pH-Bereiche auf, in denen sie jeweils ein Wirkungsoptimum besitzen; diese pH-Wertbereiche sind dem Fachmann notorisch bekannt.
Um eine optimale Enzymaktivität zu gewährleisten und die besten Ergebnisse zu erreichen, wird in Abhängigkeit der Dosierungsmenge (im Idealfall hatten sich Dosierungen von 0,01 bis 0,5% herausgestellt) und Hydrolyse-Temperatur (Optimum 45 - 55°C), eine Hydrolysezeit von 10 - 120min. variiert.
In einer weiteren Ausführungsform hat sich vorteilhaft der Zusatz von Enzymen in veganen Milchalternativen bei deren pflanzliche Proteine zur Herstellung eingesetzt wurden, herausgestellt. Neben zeitlichen und prozesstechnischen Vorteilen bei der Herstellung ergeben sich Verbesserungen in der Konsistenz, Mundgefühl, Farbe und Geschmack.

### Verfahrensdurchführung

Zum Abbau der Proteine wird die wässrige Phase mit den Mikroorganismen und/oder Enzymen versetzt. Die Protolyse erfolgt vorzugsweise in einem Rührbehälter mit kontinuierlichem Zulauf und Ablauf sowie einer Dosiervorrichtung zur Zugabe der Mikroorganismen bzw. der Enzyme und einem am Boden des Reaktors befindlichen Ventil zum Ablassen desaktivierten Mikroorganismen/Enzyme, die im Laufe der Zeit sedimentieren.

Es hat sich als vorteilhaft erwiesen, beispielsweise eine wirksame Dosierung an Mikroorganismen im Bereich von etwa 0,01 bis etwa 0,1% einzustellen.

Im Fall, dass Enzyme für die Fermentation eingesetzt werden, sollte die Konzentration vorzugsweise bei etwa 180.000 bis 250.000 FCC-Einheiten Protease pro kg Protein liegen. Mit der Dosierungsmenge kann die Aktivität und Fermentationszeit in Kombination der Temperatur variiert werden. Bspw. kann bei einer höheren Dosierungsmenge die Fermentationszeit verkürzt werden. Im Idealfall hatten sich Dosierungen von 0,01 bis 0,5% herausgestellt.

Temperatur und pH-Wert richten sich nach dem Optimum der jeweils eingesetzten Mikroorganismen bzw. Enzyme und können daher stark variieren. In der Regel wird die Fermentation aber bei einer Temperatur im Bereich von etwa 20 bis etwa 55 °C und/oder einem pH-Wert im Bereich von etwa 4,5 bis etwa 7,0 sowie über einen Zeitraum von 5 bis 24 Stunden durchgeführt. Falls erforderlich, können die Mikroorganismen bzw. Enzyme durch eine Erhitzung von 70 - 95°C für 4 bis 600 sek. und/oder eine Verschiebung des pH-Wertes inaktiviert werden.

An die Fermentation kann sich ein Reinigungs- und/oder Konzentrierungsschritt anschließen. Ferner ist es möglich die Fermentation mehrstufig, insbesondere zweistufig durchzuführen, beispielsweise in der ersten Stufe mit Mikroorganismen und in der zweiten mit Enzymen oder umgekehrt. Auch der konsekutive Einsatz unterschiedlicher Mikroorganismen oder Enzyme ist möglich. Zwischen den Fermentationsstufen können wiederum Reinigungs- und/oder Konzentrierungsschritte eingefügt werden. Anschließend kann das so erhaltene Fermentierungsprodukt den veganen Milchersatzprodukten in Mengen von beispielsweise etwa 5 bis etwa 20 Gew.-% und vorzugsweise etwa 10 bis etwa 15 Gew.-% zugesetzt werden.

In einer weiteren alternativen Ausführungsform des Verfahrens können die Mikroorganismen oder Enzyme bei der Herstellung oder kurz vor der Abfüllung der veganen Milchersatzprodukte zugegeben werden. Wahlweise könnte die Funktionalität bzw. Aktivität durch einen weiteren Prozessschritt in Form einer Temperatur-, Druckbehandlung oder ähnliches vor der Abfüllung oder in der Endverpackung des Endprodukts gestoppt werden.

### GEWERBLICHE ANWENDBARKEIT

Ein weiterer Gegenstand der Erfindung betrifft die Verwendung der fermentierten Pflanzenproteine oder des wie oben beschriebenen Verfahrenserzeugnisses zur Herstellung veganer Milchersatzprodukte, denen sie in Mengen von beispielsweise etwa 5 bis etwa 20 Gew.-% und vorzugsweise etwa 10 bis etwa 15 Gew.-% zugesetzt werden können. Unter dem Begriff "vegane Milchersatzprodukte" sind vegane Lebensmittel zu verstehen, die Milchprodukte hinsichtlich Geschmacks, Textur und Farbe nachahmen und die ausschließlich pflanzliche Komponenten enthalten, wie beispielsweise Pflanzenfette und Pflanzenfasern. Beispiele sind vegane Milch (Hafermilch, Erbsenmilch, Sojamilch), veganer Käse, veganer Joghurt und vegane Süßspeisen.

Ebenfalls beansprucht werden vegane Milchersatzprodukte enthaltend die fermentierten Pflanzenproteine oder das Verfahrenserzeugnis sowie ein Verfahren zu ihrer Herstellung, umfassend die folgenden Schritte:
(i) Bereitstellen einer veganen Milchersatzstoffgrundlage
(ii) Bereitstellen der fermentierten Pflanzenproteine oder des entsprechenden Verfahrenserzeugnisses; und
(iii) Vermischen der Komponenten (i) und (ii).

### BEISPIELE

### Beispiel 1

### Herstellung fermentierter Erbsenproteine

Es wurden 9,5kg Wasser auf 55 - 60°C erhitzt. Anschließend wurden 10kg Pflanzenfett welches bei 60 - 65°C temperiert wurde und 1,2 kg Erbsenproteine mittels einer Dispergieranlage bei einer Drehzahl von 1800U/min. dispergiert. Dabei wurde eine Trockenmasse von 7 Gew.-% eingestellt. Anschließend wurde die Erbsenprotein-Pflanzenfett-Wassermischung auf 95°C erhitzt für 5 - 10min heißgehalten und auf 40 - 45°C abgekühlt. Nach Erreichen der Kühltemperatur wurde der Mischung 10 g thermophile Säuerungskultur hinzugegeben.

Durch die Mikroorganismen der Säuerungskultur wurde nach 24h bei 40 - 45°C ein pH-Wert von 4,5 erreicht. Die durch die Fermentation geronnene beige Mischung wurde daraufhin aufgerührt und wurde zur Weiterverarbeitung in veganen Milchalternativen eingesetzt. Durch den Einsatz eines Substrats in Form von bspw. Dextrose kann die Säuerungsaktivität beschleunigt und je nach Zielsetzung weiter gesenkt werden. Dieses Ferment mit aktiven Mikroorganismen kann so zur Weiterverarbeitung in Milchalternativen eingesetzt werden um durch die aktive Kultur weitere Funktionen wie bspw. Konsistenz, Farbgebung und Aromabildung bzw. Beseitigung von negativen Proteinaromen zu beeinflussen. Denkbar wäre vor der Weiterverarbeitung auch eine Inaktivierung der Mikroorganismen durch Erhitzung auf 85°C und 5min. Heißhaltung.

### Beispiel 2

### Herstellung fermentierter Ackerbohnenproteine

Bei einer weiteren Möglichkeit wurde 28,5kg Wasser auf 55°C angewärmt. Wahlweise kann in das Wasser noch eine Zugabe von Salzen wie bspw. Natriumchlorid, etc. oder eines Substrats wie bspw. Dextrose erfolgen, welches über eine Inlinedispergierung dispergiert werden kann. Anschließend wurde eine ebenfalls auf 50 - 60°C angewärmte 30kg Pflanzenfettmischung sowie 3,6kg Ackerbohnenprotein über die Inlinedispergierung vorgemischt. Es wird somit eine Trockenmasse von 7 Gew.-% eingestellt. Anschließend wird diese Mischung auf 70°C erhitzt und über einen Hochdruckhomogenisator zweistufig 200/50bar homogenisiert. Nach der Homogenisierung erfolgt die Hocherhitzung indirekt auf 127°C mit einer Heißhaltung von 4 Sekunden mit anschließender Kühlung auf 40°C.

Hiernach wird diese vorbehandelte Mischung mit 0,05% mesophiler Kultur beimpft und für 24h bei 40°C bis zu einem pH von 4,5 fermentiert. Nach Erreichen des pH-Werts wird das Ferment aufgerührt und für die Weiterverarbeitung bereitgestellt. Hier könnte eine Aufkonzentrierung mittels Ultrafiltation erfolgen, hierdurch würde das Konzentrat farblich heller. Das konzentrierte Ferment würde zur Weiterverarbeitung zu veganen Milchalternativen bereitgestellt werden.

### Beispiel 3

### Herstellung fermentierter vegane Käsealternative mit pfl. Proteinen

Für die Fermentation einer veganen Käsealternative werden alle Zutaten wie Salze, Stabilisatoren, Stärke, Emulgatoren, pflanzliche Fette, pflanzliche Proteine, Farbstoffe und Aromen über eine Dispergierungsanlage vorgemischt, pasteurisiert und auf 40 - 45°C gekühlt. Danach wird die Kulturlösung, welche in 30°C angewärmter, pasteurisierter Kochsalzlösung (bspw. 0,09%) bereits vorbereitet wurde, in die Käsemasse aseptisch eingemischt. Anschließend erfolgt die Abfüllung unter aseptischen Bedingungen in formgebende Beutel. Nach einer Fermentationszeit von 24h bei 30 - 40°C ist der pH auf 4,5 abgesenkt. Hiernach erfolgt die Kühlung auf 6°C und nach weiteren 24 - 48h ist die vegane Käsealternative so verarbeitet, dass sie die Eigenschaften eines Feta entspricht.

### Beispiel 4

### Herstellung fermentierter Joghurtalternative mittels Enzymen

Es werden 3,5kg Erbsenprotein in 56,5kg Wasser dispergiert. Im Folgenden wird die Mischung vorerst auf 85°C erhitzt bevor diese auf 55°C gekühlt und in Fermentationsbehälter überführt wird. Im nächsten Schritt erfolgt die Zugabe von Proteasen bei einer Dosierung von 0,01 bis 0,5% auf den Proteinanteil. Die Hydrolyse verläuft über einen Zeitraum von 10 -120 Minuten bei eingeschaltetem Rührwerk. Nachfolgend wird das Produkt für 10 Minuten auf 95°C erhitzt, um die Enzyme zu inaktivieren. Abschließend erfolgt eine Kühlung auf 8°C.

Zur Weiterverarbeitung werden die Trockenstoffe in die enzymbehandelte Ausgangsbasis gegeben. Anschließend wird die Masse angewärmt bevor das bevorzugte Fett hinzugegeben wird. Im weiteren Verlauf wird das Produkt vorgewärmt bevor eine zweistufige Homogenisierung erfolgt. Nachfolgend wird die Masse auf 95°C erhitzt und im Nachhinein auf 41°C gekühlt. Im nächsten Schritt werden die Kulturen zugegeben und das Produkt fermentiert für ca. 16 Stunden bei 40°C. Abschließend wird die Joghurtalternative auf 85°C erhitzt bevor diese auf 30°C gekühlt und abgefüllt wird.

### Anwendungsbeispiel

Es wurde ein veganer Käse jeweils unter Einsatz der fermentierten und unfermentierten Proteine hergestellt und die sensorischen Eigenschaften anschließend jeweils auf einer Skala von (1) = schwach bis (5) = stark ausgeprägt beurteilt. Die Ergebnisse sind in Tabelle 1 wiedergegeben. Die Beispiele 1 und 2 sind erfindungsgemäß, die Beispiele V1 und V2 dienen zum Vergleich.

**Tabelle 1**

| Zusammensetzung veganer Käse - Mengenangaben als Gew.-% | | | | |
|---|---|---|---|---|
| **Komponente** | **V1** | **1** | **V2** | **2** |
| Kokosfett | 20,0 | 20,0 | 20,0 | 20,0 |
| Sheabutter | 4,0 | 4,0 | 4,0 | 4,0 |
| Haferprotein (unfermentiert) | 5,0 | - | - | - |
| Haferprotein Bsp. 1 | - | 5,0 | - | |
| Lupinenprotein (unfermentiert) | - | - | 5,0 | - |
| Lupinenprotein Bsp. 2 | - | - | - | 5,0 |
| Salz | 1,5 | 1,5 | 1,5 | 1,5 |
| Carrageen | 0,5 | 0,5 | 0,5 | 0,5 |
| Stärke | 24,5 | 24,5 | 24,5 | 24,5 |
| Dextrose | 0,5% | 0,5% | 0,5% | 0,5% |
| Aromastoffe | 0,5 | 0,5 | 0,5 | 0,5 |
| Wasser | Zu 100 Gew.-% | | | |

| ***Eigenschaften der Proteine*** | | | | |
|---|---|---|---|---|
| Farbhelligkeit | 3 | 5 | 3 | 5 |
| Bittergeschmack | 4 | 1 | 5 | 2 |
| Säurenote im Geruch | 1 | 3 | 1 | 3 |

| ***Eigenschaften des Endproduktes*** | | | | |
|---|---|---|---|---|
| Cremigkeit | 2 | 5 | 2 | 5 |
| Mundgefühl | 2 | 5 | 2 | 5 |
| Klebrigkeit | 2 | 1 | 3 | 1 |

Im Vergleich mit den unfermentierten Produkten zeigen die fermentierten Pflanzenproteine eine hellere Farbe, praktisch keinen Bittergeschmack sowie eine angenehme säuerliche Geruchsnote. Die erfindungsgemäßen Endprodukte sind cremiger, weniger klebrig und besitzen ein höheres Mundgefühl als die Vergleichsprodukte.

## Patentansprüche

1. Fermentierte Pflanzenproteine, erhältlich oder erhalten durch die folgenden Schritte
(a) Bereitstellen einer pflanzlichen Proteinquelle;
(b) Versetzen der Proteine aus Schritt (a) mit Wasser unter Erhalt einer wässrigen Proteinphase;
(c) Versetzen der wässrigen Proteinphase aus Schritt (b) mit Mikroorganismen, die zur Fermentation von Proteinen geeignet sind, und/oder Proteasen; und
(d) Fermentierung der wässrigen Proteinphase aus Schritt (c) sowie gegebenenfalls
(e) Inaktivierung und Abtrennung der Mikroorganismen und/oder Enzyme.

2. Verfahren zur Herstellung von fermentierten Pflanzenproteinen umfassend oder bestehend aus den folgenden Schritten:
(a) Bereitstellen einer pflanzlichen Proteinquelle;
(b) Versetzen der Proteine aus Schritt (a) mit Wasser unter Erhalt einer wässrigen Proteinphase;
(c) Versetzen der wässrigen Proteinphase aus Schritt (b) mit Mikroorganismen, die zur Fermentation von Proteinen geeignet sind, und/oder Proteasen; und
(d) Fermentierung der wässrigen Proteinphase aus Schritt (c) sowie gegebenenfalls
(e) Inaktivierung und Abtrennung der Mikroorganismen und/oder Enzyme.

3. Verfahren nach Anspruch 2, **dadurch gekennzeichnet, dass** man Pflanzenproteine einsetzt, die ausgewählt sind aus der Gruppe, die gebildet wird von Ackerbohnenproteinen, Haferproteinen, Reisproteinen, Mandelproteinen, Eichelproteinen, Kartoffelproteinen, Sojaproteinen, Erbsenproteinen, Lupinenproteinen, Rapsproteinen Sonnenblumenproteinen und Kürbiskernproteinen sowie deren Mischungen.

4. Verfahren nach den Ansprüchen 1 und/oder 2, **dadurch gekennzeichnet, dass** man Pflanzenproteine einsetzt, die zuvor einer Druck-Temperatur-Vorbehandlung unterzogen worden sind.

5. Verfahren nach mindestens einem der Ansprüche 2 bis 4, **dadurch gekennzeichnet, dass** man der wässrigen Proteinphase aus Schritt (b) mindestens ein Pflanzenfett und/oder mindestens einen Ballaststoff zusetzt.

6. Verfahren nach mindestens einem der Ansprüche 2 bis 5, **dadurch gekennzeichnet, dass** man Pflanzenfette einsetzt, die ausgewählt sind aus der Gruppe, die gebildet wird von Kakaobutter, Kokosöl, Palmöl, Palmkernöl, Kokosfett, Nussöl, Sheabutter, Aprikosenöl, Sojalecithin, Sonnenblumenöl, Sojaöl, Rapsöl, Nussöl sowie deren Gemischen.

7. Verfahren nach mindestens einem der Ansprüche 2 bis 6, **dadurch gekennzeichnet, dass** man als Ballaststoffe Pflanzenfasern einsetzt.

8. Verfahren nach mindestens einem der Ansprüche 2 bis 7, **dadurch gekennzeichnet, dass** man Mikroorganismen einsetzt, die ausgewählt sind aus der Gruppe der mesophilen und thermophilen Säurebildner, die gebildet wird durch den Stoffwechsel mit also auch ohne Einsatz eines Substrats.

9. Verfahren nach mindestens einem der Ansprüche 2 bis 8, **dadurch gekennzeichnet, dass** man Enzyme einsetzt, die ausgewählt sind aus der Gruppe, die gebildet wird von Pepsin, Chymosin, Cathepsin E, Papain, Cathepsin K, Caspase, Calpain), Chymotropsin, Plasmin, Thrombin, Trypsin, Granzyme, Kallikrein und Proteasom sowie deren Mischungen.

10. Verfahren nach mindestens einem der Ansprüche 2 bis 9, **dadurch gekennzeichnet, dass** man die Fermentation bei einer Temperatur im Bereich von etwa 30 bis etwa 55°C und/oder einem pH-Wert im Bereich von etwa 3,5 bis etwa 7,0 durchführt.

11. Verfahren nach mindestens einem der Ansprüche 2 bis 10, **dadurch gekennzeichnet, dass** sich an die Fermentation ein Reinigungs- und/oder Konzentrierungsschritt anschließt.

12. Verfahren nach mindestens einem der Ansprüche 2 bis 11, **dadurch gekennzeichnet, dass** die Fermentation mehrstufig durchgeführt wird.

13. Verwendung der fermentierten Pflanzenproteine nach Anspruch 1 oder des Verfahrenserzeugnisses nach mindestens einem der Ansprüche 2 bis 12 zur Herstellung veganer Milchersatzprodukte.

14. Vegane Milchersatzprodukte enthaltend die fermentierten Pflanzenproteine nach Anspruch 1 oder das Verfahrenserzeugnis nach mindestens einem der Ansprüche 2 bis 12

15. Verfahren zur Herstellung von veganen Milchersatzprodukten, umfassend die folgenden Schritte:
(i) Bereitstellen einer veganen Milchersatzstoffgrundlage
(ii) Bereitstellen der fermentierten Pflanzenproteine nach Anspruch 1 oder des Verfahrenserzeugnisses nach mindestens einem der Ansprüche 2 bis 12; und
(iii) Vermischen der Komponenten (i) und (ii).
